# EUROPEAN PATENT SPECIFICATION

(11) **EP 1 870 037 B1**
(45) Date of publication and mention of the grant of the patent: **04.11.2009**
(21) Application number: 06425414.7
(22) Date of filing: 19.06.2006
(51) Int. Cl.: A61B 5/11, G08B 21/04

(54) **Apparatus and method of detecting falls and immobility**
Vorrichtung und Verfahren zur Erkennung von Stürzen und Unbeweglichkeit
Dispositif et méthode de détection des chutes et d'immobilité

(43) Date of publication of application: 26.12.2007
(73) Proprietor: Universita' degli Studi di Bari, 70121 Bari (IT)
(72) Inventor: Quagliarella, Livio c/o Policlinico di Bari, I-70126 Bari (IT); Sasanelli, Nicola c/o Policlinico di Bari, I-70124 Bari (IT); Belgiovine, Giuseppe c/o Policlinico di Bari, I-70124 Bari (IT); Cutrone, Natalizia c/o Politecnico di Bari, Campus Universitario I-70126 Bari (IT)
(74) Representative: Valentini, Giuliano

(56) References cited:
- EP-A- 1 575 010
- US-A1- 2002 116 080
- US-B1- 6 544 200
- US-B2- 6 433 690

## Description

The present invention relates to a portable apparatus, particularly of the type to be worn, for detecting falls and subsequent faints in subjects, and a method for carrying out this detection.

Portable apparatuses have been available on the market for a long time, which allow a subject to send a call for help in case of need, such as due to a sudden indisposition, accidental fall, or generally, due to an undesired alteration of his/her own physical-psychical state. These apparatuses are particularly widespread in the geriatric field, for remote assistance to elderly people, in the therapeutic field, for assistance to either handicapped or people undergoing physical rehabilitation, in the sports field, for signalling indispositions, etc.

A known traditional apparatus, which is available from BEGHELLI under the name of "Telesalvalavita Beghelli", comprises a fixed central unit that can be connected to a home phone line, and a portable remote control. The fixed unit comprises a memory in which the phone numbers are stored, which have to be called in case of need. By pressing a key on the remote control, the user activates the fixed unit, which sequentially selects the number stored therein and sends a pre-recorded call for help.

The traditional apparatuses to be activated by the user, i.e. with voluntary call, have a series of drawbacks. First, as the activation of the apparatus for sending the call for help is relied on the user, the same apparatus is useless if the user faints or cannot use the remote control, such as because of motor problems resulting from either a fall or an indisposition.

In many application, however, it is required to be able of having an apparatus capable of sending a call for help even when the subject cannot activate it.

A further drawback is due to the fact that the remote control works only within a predetermined receiving range. If the subject moves to a greater distance, the fixed unit is not capable of receiving the signal generated by the remote control as it is activated, thereby resulting in the apparatus not sending out the call for help.

The remote control can be easily activated in an inadvertent manner. The remote control is usually provided with a large-sized activation button, which can be easily reached also by persons suffering from motor problems. The remote control is usually worn by the subject around the neck, and due to the size of the activation button, the latter can be inadvertently pressed, and the fixed unit is activated without a real need for help, with clear negative consequences in terms of resource management.

Another type of portable apparatuses provides that the subject is continuously monitored.

An apparatus of the type to be worn, developed by "VA Palo Alto Rehabilitation R&D Center" and called "WAMAS" (Wearable Accelerometric Motion Analysis System), allows monitoring the wearer's movements by means of a plurality of sensors. Some sensors are positioned on a belt, others on the frame of a pair of glasses that the user must wear. The apparatus detects the accelerations of the subject's pelvis and the accelerations of his/her head. The data detected can be used for analyzing the subject's movement for either medical or sports application, or for help in case of fall. The detection is operated in a continuous manner, i.e. the apparatus is always active and constantly records the data detected.

Disadvantageously, the apparatus provides the use of glasses. Furthermore, the apparatus comprises expensive and complex means for acquiring and processing the data detected by the sensors, such as processors, computers, mass memories (such as hard disks), etc. In other words, the apparatus is not comfortable to wear, complicated to develop and expensive. A further drawback is due to the fact that the continuous operation requires using heavy-duty batteries, i.e. heavy and/or bulky batteries.

The European Patent Application EP-A-0846440, in the name of SARCOS, INC., relates to an apparatus to be worn for continuously monitoring the physical conditions and the position of a soldier on a battle field. This apparatus, which is intended for use in a military environment, and hence, under severe conditions, is complex to make and develop, and particularly expensive. The apparatus comprises a plurality of sensors arranged on a waistcoat to be worn by a soldier. The sensors can be temperature (of either the body and/or environment) sensors, accelerometers, GPS detectors, cardiac sensors, etc. The signals acquired by the sensors are processed by a suitable control unit and/or sent to a remote unit, such as the headquarters or a doctor. Thereby, it is possible to ascertain whether the soldier has fainted or is injured. The apparatus further comprises a display allowing the soldier to visualize the parameters being monitored and receive an estimate of his own physical conditions based on these parameters. Means for carrying out injections, when needed, can be installed in the waistcoat. The apparatus controls these means for dosing the medicaments to be injected based on the diagnosis issued.

It is understood that this type of apparatus is intended for a specific use in the military field and is hardly adapted for use in different fields. Besides being particularly expensive, this apparatus suffers from the drawbacks that have been already mentioned for the WAMAS apparatus in relation to the continuous monitoring of the subject's physical conditions.

EP-A1-1575010 discloses a device to monitor a set of vital parameters of a user and send an alarm call when the values of said parameters exceed the normal threshold values, thus indicating a potential situation of danger. Among these situations, also a possible fall and faint condition can be detected by a bi-axial accelerometer with sensing axes X and Y parallel to the floor in order to detect the mobility of the user. However, this document is substantially silent about the processing of the signal generated by the accelerometer in the case of falls.

US-A1-2002/0116080 discloses a monitoring device having a three-axis accelerometer for detecting possible falls of a user. The detected signals are compared to stored threshold values in order to determine a possible fall condition of the user.

The need has been felt for a long time to have a portable apparatus for detecting falls and/or faints which is simple to make and use, cost-effective and not bulky, i.e. such as to be used in civil applications and widely spread. The object of the present invention is to provide a portable apparatus for detecting falls and/or faints of subjects, which solves the drawbacks of the traditional apparatuses in a simple though effective manner.

It is also an object of the present invention to provide a portable apparatus for detecting falls and/or faints in subjects, which does not necessarily requires to be activated by the subject in the event he/she has to be helped.

It is still another object of the present invention to provide a portable apparatus for detecting falls and/or faints in subjects, which is cost-effective and simple to make and develop.

It is another object of the invention to provide a portable apparatus for detecting falls and/or faints in subjects, which is simple to be used also by old, handicapped persons, or persons suffering from motor dysfunctions, etc.

It is yet another object of the present invention to provide a simple and effective manner for generating an alarm signal following a fall or faint of a subject.

These and other objects are achieved by the present invention, which relates to a portable apparatus according to claim 1.

Advantageously, the apparatus according to the present invention provides that the call for help is sent when the subject is not in the condition of manually controlling the apparatus.

The apparatus sends a call for help to the remote control station for helping the subject even when the latter has fainted or is not self-sufficient.

The apparatus can be worn, such as by being attached to a belt. The sensor detecting the accelerations is an accelerometer, preferably of a biaxial type, i.e. capable of detecting the accelerations about/along two axes, of the portions of the subject's body to which it is applied.

The sensor generates a signal indicative of the accelerations of the subject's body. The control unit, such as a processor, compares the trend of the accelerations measured over time by the sensor with preset default trends, such as stored in the same control unit, and based on this comparison, it checks whether the subject has fallen and if, after the fall, he/she remains motionless. Preferably, the control unit detects that threshold conditions relative to (synthetic) parameters calculated based on the detected signals has been exceeded. For example, the control unit detects the number of peaks of the accelerations as measured by the sensor and estimates whether this number exceeds a reference value, whether the peaks are followed by a rotation of the reference axis of the sensor and whether they are followed by null or minimum accelerations.

The condition in which a number of acceleration peaks (such as five) is detected in the time unit (such as 1 second), followed by a rotation of the axis of measurement of the sensor and a prolonged absence of accelerations, is indicative of a fall.

The control unit also functions as a timer for measuring the time elapsing from when the fall is identified by the control unit, i.e. the time in which the comparison on the trends of the (measured and stored) accelerations gives a positive result. After a first predetermined time interval, such as 60 seconds, a pre-alarm signal is generated to inform the subject that the apparatus will provide to send a call for help.

The pre-alarm signal is generated by a suitable signaller, which can be acoustic, luminous, or a vibrating element, controlled by the control/timer unit. The pre-alarm signal triggers the "timer" function integrated within the control unit for counting a second time interval, such as 30 seconds, within which the subject can deactivate the apparatus or however prevent the call for help from being sent out. Thereby, the subject, who is suitably warned by the signaller that the call for help is going to be sent out, has the chance of stopping this transmission if his/her physical conditions do not require any external intervention, i.e. if the subject believes he/she does not need any help.

In other words, the apparatus according to the present invention provides sending the call automatically to a remote station for help, and voluntary blocking the call by the subject in order to prevent the call from being carried out when the subject does not deem it necessary.

This characteristic is particularly advantageous when the equipment is used in the sports field, such as for climbing, cycling, run, etc., and generally for those activities in which the athletes often fall but without particular physical consequences, and in the medical field, for the assistance to elderly people suffering from deambulation problems or handicapped people with motor problems, though however self-sufficient, etc.. As the subject is allowed to stop the call for help, "false alarms" can be avoided, i.e. the help operators are prevented from intervening when not required, based on the subject's judgement, with clear advantages in terms of management of the resources engaged in the help operations.

For example, the apparatus can be provided with a button, which the subject has to press voluntarily in order to avoid sending the call for help, or, when the call for help has been sent, in order to communicate that help is not required (a second call is sent to cancel the first one). In other words, the subject can stop the call for help from being sent, or if the latter has been sent, communicate that the help is actually unnecessary.

At the end of the second time interval, as measured by the timer, if the subject does not provide to press the block button, for example because he/she is fainted following the fall, the apparatus sends out the call for help.

The communication means preferably comprise an interface for connection to the GSM phone line. Thereby, the apparatus does not suffer from the problems of the prior art apparatuses with reference to the range, on the contrary, it is operative everywhere a GSM phone network coverage is provided.

According to a first embodiment of the apparatus, the remote help station can re-call the subject, such as through the GSM phone line, after a call for help has been sent. The operator of the remote help station can thus interact with the subject to check whether the subject is conscious and his/her physical conditions actually require any help.

Preferably, the apparatus is further provided with a button for voluntary call by the subject. This button has a small size and is positioned such that the risk of being unintentionally operated is minimized. By pressing the voluntary call button, the subject sends a call for help.

The present invention further relates to a method for sending a call for help according to claim 9.

The apparatus can be provided with an uncoupling sensor having the function of detecting when the apparatus is removed by the subject, such as when the subject takes off his/her belt on which the apparatus is mounted. Thereby, the apparatus is prevented from causing a false alarm when it is left still. The uncoupling sensor sends a suitable signal to the control unit.

As an alternative to the uncoupling sensor, or in addition thereto, the apparatus can be provided with a temperature sensor suitable to detect the subject's body temperature. The temperature sensor sends the relative signal to the control unit, which based on this signal, can estimate whether the apparatus is in contact with the subject's body, i.e. it is operating, or not.

The calibration of the apparatus provides that one or more typical acceleration trends of those subjects falling from a still position or while walking, etc., are stored within the control unit, or that threshold values of parameters concerning the magnitudes being measured are stored. A software is installed in the control unit in order to provide the instructions required either to carry out the comparison between the trends stored and the trends detected by the accelerometer, or in order to detect that threshold values concerning the acquired signals have been exceeded.

The signal can be processed by the control unit both in real time, and when the apparatus is in a stand-by condition.

Advantageously, the apparatus and the method according to the present invention does not necessarily require to be voluntarily activated by the subject in the event that help is required.

This characteristic allows also non self-sufficient persons to be effectively assisted, or however those people who are not capable of voluntarily activating a traditional apparatus, following a fall. Furthermore, the apparatus and method according to the present invention also allows those subjects who have fainted and that remain still for a preset interval of time to send a call for help.

The apparatus according to the present invention has a simple structure and can be made with a small number of electrical/electronic components that are available on the market with a reasonable price. Furthermore, the apparatus is user-friendly also to elderly people, handicapped people, or suffering from motor dysfunctions, etc., since it works in an automatic manner. The apparatus does not require using glasses or other appendixes, but on the contrary is compact and can be contained in a small-sized housing, that can be fixed to a belt to be worn by the subject.

Unlike the apparatus according to EP-A-0846440, the apparatus according to the present invention does not communicate with the control station in a continuous manner, i.e. it does not allow monitoring the subject's physical conditions from a remote site. The apparatus according to the invention communicates with the remote station only when it sends the call for help and, therefore, the power source supplying the apparatus can have minimum bulk and capacity. For example, when the apparatus is battery-powered, the latter has a small size and is thus lightweight.

Further aspects and advantages of the present invention will be understood more clearly from the description below, which is given by way of non-limiting illustration with reference to the annexed schematic drawings, in which:
- Fig. 1 is a base drawing of the method according to the present invention;
- Fig. 2 is a schematic view of an apparatus according to the present invention;
- Fig. 3 is a first diagram concerning the operation of the apparatus shown in Fig. 1;
- Fig. 4 is a second diagram concerning the operation of the apparatus shown in Fig. 1;
- Fig. 5 is a third diagram concerning the operation of the apparatus shown in Fig. 1.

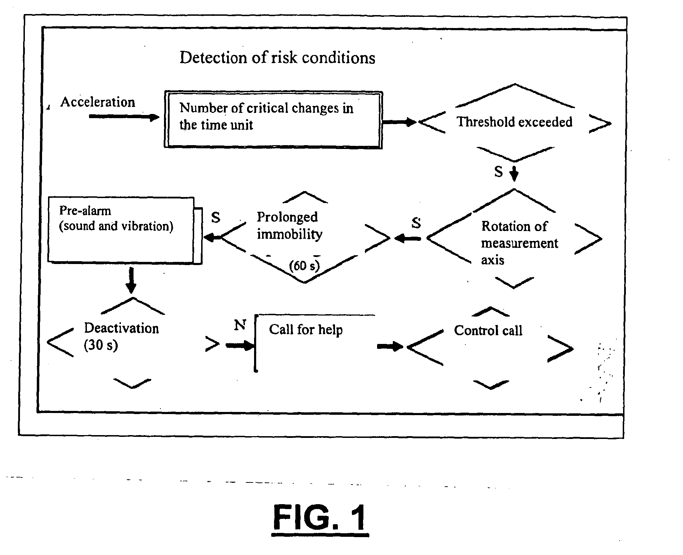

With reference to Fig. 1, the apparatus according to the present invention comprises a sensor detecting the acceleration of at least one portion of the subject's body. The sensor sends a signal indicative of the acceleration measured to a control unit. The control unit compares this input signal with reference parameters stored therein.

Particularly, the control unit compares the trends of the acceleration, as measured by the sensor over time, with reference trends, in order to recognize an acceleration corresponding to a fall of the subject provided with the apparatus. The comparison or processing also provides useful information for identifying whether the subject has fainted.

Preferably, the control unit MCU is programmed to process the acceleration signal provided by the sensor and detect the number of peaks that, in the time unit, exceed a predetermined threshold value. The threshold value can be equal to 1,2 g, for example. If the number of peaks detected by the unit MCU exceeds a preset number, the unit MCU itself provides to establish whether the sensor has undergone a rotation. The sensor, which is preferably of the biaxial type, is sensitive to the rotation of its own measurement axis. The control unit is capable of detecting this rotation, which is indicative of a possible fall of the subject. If, following the rotation of the sensor, the control unit detects a null or substantially null acceleration over a prolonged time interval, which situation indicates that the subject is motionless, the control unit MCU itself provides to activate the pre-alarm signal.

When the control unit, based on said comparison/processing operation, recognizes that the subject has fallen, provides to send a call for help within a preset time interval, within which the subject can intervene in order to prevent the request from being carried out.

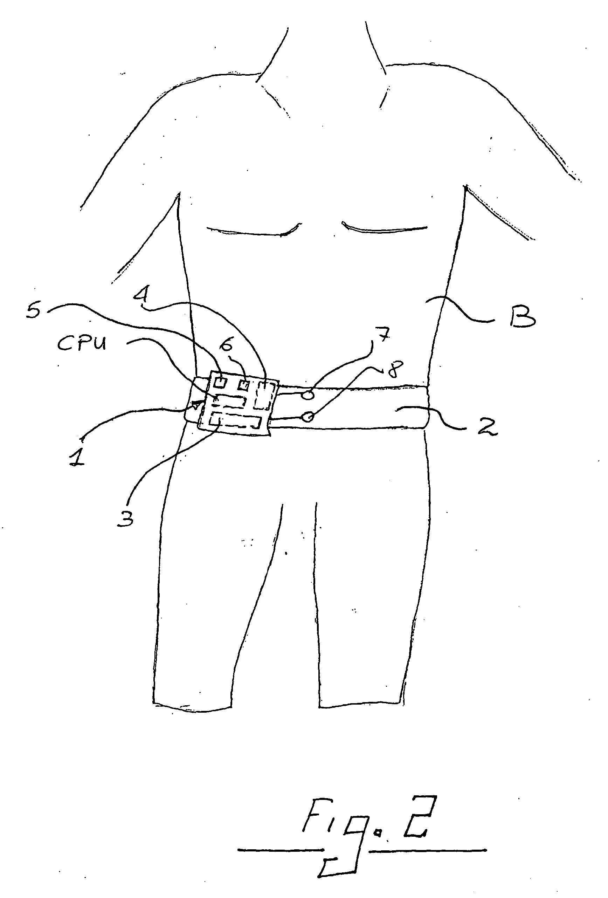

Fig. 2 shows an embodiment of an apparatus 1 according to the present invention. The apparatus 1 is enclosed in a housing that can be fastened to a belt 2 worn by the subject B.

The apparatus comprises a sensor 3, preferably an accelerometer, to detect the accelerations of the body of subject B. In the embodiment shown, the accelerometer 3 is of a biaxial type, i.e. it detects the accelerations along/about two Cartesian axes, particularly the accelerations of the pelvis of subject B.

The signal generated by the sensor 3 is sent to a control unit MCU for processing. The control unit MCU is programmed to process the signal provided by the sensor 3 according to algorithms for comparison with the data stored in the same unit MCU.

Particularly, the sensor 3 generates a signal indicating the accelerations of subject B over time. In the control unit MCU there are stored the typical trends of a subject's accelerations during his/her normal daily activities, such as walking, running, sitting, etc..

The control unit MCU compares the signal provided by the sensor 3 with these trends stored therein, in order to detect any difference indicating that subject B has fallen and that he/she is subsequently motionless, such as due to a faint. Particularly, the control unit MCU is programmed for:
- counting the number of acceleration peaks which exceed a determined peak threshold value;
- detecting the rotation of the measurement axis of the biaxial acceleration sensor 3;
- detecting the subsequent prolonged immobility of subject B (acceleration signal substantially equal to zero).

When the control unit MCU, based on said comparison, detects that the subject B has fallen, it starts counting the time elapsing from the fall and during which the subject B is motionless. This function is performed by the control unit MCU.

The processing and comparison can be carried out by the control unit MCU both in real time, and when the apparatus is in a stand-by condition.

After a first preset time interval as calculated by the timer/MCU, for example 60 seconds, the control unit MCU activates a signaller 5, which generates a pre-alarm signal to inform the user B that the apparatus 1 is going to send a call for help to the relative remote control unit.

The signaller 5 can generate either a luminous or sound signal, or it can vibrate. The timer MCU counts a second time interval, such as equal to 30 seconds, starting from the pre-alarm signal. During this second interval, the subject B, through suitable means of the apparatus 1, can prevent the call for help from being sent. For example, the apparatus 1 can be provided with a button 6 for deactivation.

If, at the end of the second time interval, as measured by the timer MCU, the subject does not provide to press the button 6, for example because he/she has fainted following the fall, the apparatus 1 sends the call for help.

In order to avoid false alarms (such as the subject B is not capable of pressing the button 6 in time), the subject B can send a second call to the remote control station, such as via the same button 6, in order to cancel the first call, i.e. to indicate that help is not necessary even though the apparatus 1 has provided to send out the call.

The apparatus 1 communicates with the remote station by means of suitable communication means (not shown). Preferably, these means comprise an interface for connecting the control unit MCU to the GSM phone network.

The apparatus 1 preferably further comprises a temperature sensor 7 having the function of detecting the temperature at the body of subject B. If the subject B takes off the belt 2, the sensor 7 measures a temperature change, due to the fact that the apparatus is no longer in contact with the body of the subject B, i.e. it cools down. The signal of the temperature sensor 7 is used by the unit MCU to detect when the apparatus 1 is separated from the subject B and thus it is not used.

In the embodiment as shown in Fig. 2, the apparatus 1 further comprises a uncoupling sensor 8 capable of detecting that the belt 2 has uncoupled from subject B or apparatus 1 from belt 2. The signal generated by the sensor 8 is used by the MCU to detect an "unused" condition of apparatus 1.

Preferably, the apparatus 1 comprises a button (not shown) to send a voluntary call for help. The button has a minimum size and is placed such as to prevent any inadvertent activation.

The apparatus 1 can be provided with a unit 4 for detecting the position of the subject. For example, the unit 4 shown in Fig. 2 is a GPS satellite detector. The information concerning the position of the subject is sent to the remote station together with the call for help, such as to simplify external interventions.

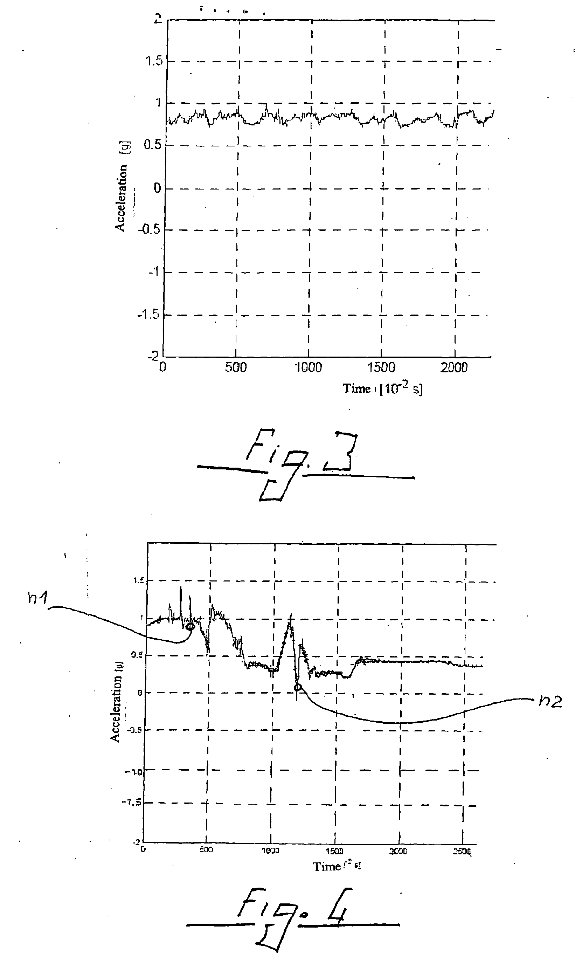

Fig. 3 is a diagram showing the acceleration signal as acquired by the sensor 3 when the subject B is walking. Acceleration peaks exceeding a predetermined threshold (in intensity) are not detected. The control unit MCU does not provide to send a call for help.

Fig. 4 is a diagram showing the acceleration signal as acquired by the sensor 3 when the subject B is carrying out a normal daily activity, before he/she takes off the belt 2 provided with the apparatus 1. The first part of the trend, ranging between 0 and 1500 [10⁻² s], concerns the normal daily activity carried out by the subject B. The control unit MCU, by processing the signal, has identified two acceleration peaks n1 and n2. In the second part of the trend, beyond 1500 [10⁻² s], the acceleration flattens, which indicates that the subject B has taken off his/her belt 2, such as by placing the same stationary on a support. The uncoupling sensor 8 sends a signal to the unit MCU to indicate that the apparatus 1 is not acquiring signals from subject B and informs the subject that the apparatus requires to be turned off, such as by means of a sound and/or luminous signal.

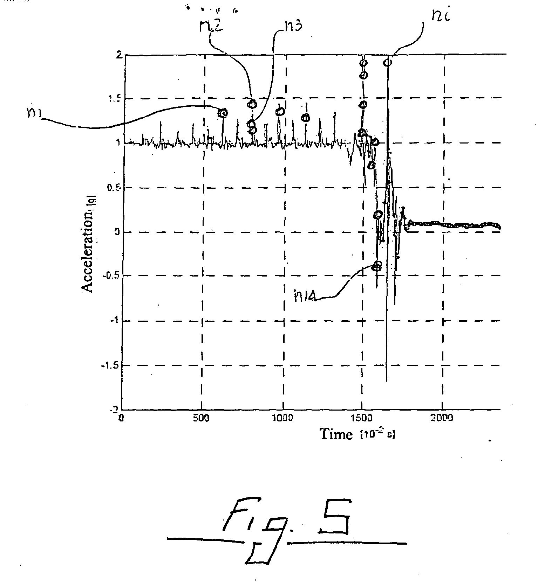

Fig. 5 is a diagram showing the acceleration signal as acquired by sensor 3 in the event that subject B falls. During the first 1700 [10⁻² s] the control unit MCU detects a plurality of acceleration peaks n1, n2, n3... , n14 the intensity of which exceeds a predetermined threshold value. After a last peak nᵢ, i.e. after the first 1700 [10⁻² s] of the acquisition time, the acceleration trend flattens and tends to zero, which indicates that the subject B is motionless. The control unit MCU is programmed to construe the sequence of a plurality of acceleration peaks n1-nᵢ, followed by an acceleration substantially equal to zero, such as a scheme indicating that the subject B has fallen and is then motionless, and provides to send the call for help in an automatic manner.

## Claims

1. A portable apparatus (1) for detecting falls and immobility of a subject (B), comprising at least one sensor (3) generating a signal indicative of the accelerations of at least one portion of the body of said subject (B), a control unit suitable to process said signal and communication means for sending a call for help to a remote control station, wherein said control unit is programmed to automatically activate said communication means only when a fall of the subject and his/her subsequent immobility are detected based on said processing, **characterized in that** said control unit is programmed to process said signal by:
- counting the number of acceleration peaks exceeding a determined peak threshold value in the time unit;
- detecting a subsequent rotation of the measure axis of said sensor (3);
- subsequently detecting a null acceleration signal for a certain time, which indicates that said subject (B) is motionless.

2. The apparatus according to claim 1, **characterized in that** it further comprises means (6) for cancelling or confirming a call for help, either before or after the call for help has been sent out.

3. The apparatus according to claim 1 or 2, **characterized in that** said control unit comprises an integrated timer detecting the time elapsed from said detection of fall.

4. The apparatus according to claim 3, **characterized in that** said communication means is capable of being deactivated by said subject in order to prevent the call for help from being sent within a preset time interval as measured by said timer.

5. The apparatus according to any preceding claim 1-4, **characterized in that** it comprises a signaller (5) generating a pre-alarm signal for said subject (B) before sending out said call for help.

6. The apparatus according to any preceding claims 1-5, **characterized in that** said sensor (3) is a bi-axial accelerometer.

7. The apparatus according to any preceding claim 1-6, **characterized in that** it further comprises one or more sensors selected from a temperature sensor (7) suitable to detect the body temperature of said subj ect and an uncoupling sensor (8) suitable to detect that the apparatus has been separated from said subject (B).

8. The apparatus according to any preceding claim 1-7, **characterized in that** the communication means comprise an interface for connection to the GSM phone network.

9. A method for sending a call for help following falls and immobility of a subject (B), comprising the steps of measuring the accelerations of at least one portion of the body of said subject, comparing said accelerations over time with reference values in order to identify a fall and immobility of the subject, and the step of sending a call for help when required based on said comparison, said call for help being sent in an automatic manner, **characterized in that** said comparison step provides:
- counting the number of acceleration peaks exceeding a determined peak threshold value in the time unit;
- detecting whether a subsequent rotation of said subject (B) has occurred
- detecting, when said rotation has occurred, whether the acceleration is null for a certain time, said automatic call for help being sent after a preset time interval from when said fall and immobility are detected based on said comparison.

10. The method according to claim 9, **characterized in that** said step of sending the automatic call for help can be manually activated by said subject or manually stoppable either during or after said preset time interval.

11. The method according to claim 9 or 10, **characterized in that** it further comprises the step of generating a pre-alarm signal to inform said subject that the call for help is going to be sent out.

12. The method according to any preceding claim 9-11, **characterized in that** it further comprises the step of measuring the temperature of the environment around said subject.

## Patentansprüche

1. Tragbare Vorrichtung (1) zur Erkennung von Stürzen und Reglosigkeit eines Subjekts (B), umfassend wenigstens einen Sensor (3) zur Erzeugung eines Signals anzeigend die Beschleunigung von wenigstens einem Teil des Körpers des Subjekts (B), eine Kontrolleinheit zur Verarbeitung des Signals und Kommunikationsmittel zum Senden eines Notrufs an eine entfernte Kontrollstation, wobei die Kontrolleinheit programmiert ist, die Kommunikationsmittel nur automatisch zu aktivieren, wenn ein Sturz des Subjekts und eine nachfolgende Reglosigkeit basierend auf der Verarbeitung erkannt wurde, **dadurch gekennzeichnet, dass** die Kontrolleinheit programmiert ist, das Signal zu verarbeiten durch:
- Zählen der Anzahl von Beschleunigungsspitzen, die einen bestimmten oberen Schwellenwert in einer Zeiteinheit überschreiten;
- Erkennen einer nachfolgenden Drehung der Messachse des Sensors (3);
- nachfolgendes Erkennen eines Null-Beschleunigungssignals für eine bestimmte Zeit, was anzeigt, dass das Subjekt (B) regungslos ist.

2. Vorrichtung nach Anspruch 1, **dadurch gekennzeichnet, dass** die Vorrichtung weiterhin Mittel (6) zum Zurücknehmen oder Bestätigen eines Notrufs umfasst, entweder bevor oder nachdem der Notruf gesendet wurde.

3. Vorrichtung nach Anspruch 1 oder 2, **dadurch gekennzeichnet, dass** die Kontrolleinheit einen integrierten Zähler umfasst, welcher die Zeit die seit dem Erkennen des Sturzes vergangen ist erkennt.

4. Vorrichtung nach Anspruch 3, **dadurch gekennzeichnet, dass** die Kommunikationsmittel durch das Subjekt innerhalb eines durch den Zähler bestimmten Zeitintervalls deaktivierbar sind, um das Senden des Notrufs zu verhindern.

5. Vorrichtung nach einem der Ansprüche 1 bis 4, **dadurch gekennzeichnet, dass** die Vorrichtung einen Signalgeber (5) umfasst, der ein Voralarmsignal für das Subjekt (B) erzeugt, bevor der Notruf ausgesendet wird.

6. Vorrichtung nach einem der Ansprüche 1 bis 5, **dadurch gekennzeichnet, dass** der Sensor (3) ein biaxialer Beschleunigungsmesser ist.

7. Vorrichtung nach einem der Ansprüche 1 bis 6, **dadurch gekennzeichnet, dass** die Vorrichtung weiterhin einen oder mehrere Sensoren umfasst, ausgewählt aus einem Temperatursensor (7), um die Körpertemperatur des Subjekts zu erkennen und einem Abkupplungssensor (8) zum Erkennen, dass die Vorrichtung von dem Subjekt (B) getrennt wurde.

8. Vorrichtung nach einem der Ansprüche 1 bis 7, **dadurch gekennzeichnet, dass** die Kommunikationsmittel eine Schnittstelle zur Verbindung mit dem GSM-Telefonnetz umfassen.

9. Verfahren zum Senden eines Notrufs nach einem Sturz und Regungslosigkeit eines Subjekts (B), umfassend die Schritte des Messens der Beschleunigung von wenigstens einem Teil des Körpers des Subjekts, Vergleichen der Beschleunigung über die Zeit mit Referenzwerten, um einen Sturz und Regungslosigkeit des Subjekts zu erkennen, und basierend auf dem Vergleich den Schritt des Sendens eines Notrufs im Bedarffall, wobei der Notruf automatisch gesendet wird, **dadurch gekennzeichnet, dass** der Vergleichsschritt umfasst:
- Zählen der Anzahl von Beschleunigungsspitzen, die einen bestimmten oberen Schwellenwert in einer Zeiteinheit überschreiten;
- Erkennen, ob eine nachfolgende Drehung des Subjekts (B) stattgefunden hat;
- Erkennen, ob die Beschleunigung für eine bestimmte Zeit 0 ist, wenn die Rotation stattgefunden hat, wobei der automatische Notruf nach einem bestimmten Zeitraum gesendet wird nach dem Erkennen des Sturzes und der Regungslosigkeit mittels des Vergleichs.

10. Verfahren nach Anspruch 9, **dadurch gekennzeichnet, dass** der Schritt des Sendens eines automatischen Notrufs durch das Subjekt aktivierbar ist oder entweder während oder nach dem bestimmten Zeitraum anhaltbar ist.

11. Verfahren nach einem der Ansprüche 9 oder 10, **dadurch gekennzeichnet, dass** das Verfahren weiterhin den Schritt des Erzeugens eines Voralarmsignals umfasst, um das Subjekt zu informieren, dass ein Notruf ausgesendet werden wird.

12. Verfahren nach einem der Ansprüche 9 bis 11, **dadurch gekennzeichnet, dass** das Verfahren weiterhin den Schritt des Messens der Temperatur der Umgebung des Subjekts umfasst.

## Revendications

1. Appareil portable (1) pour détecter les chutes et l'immobilité d'un sujet (B), comprenant au moins un capteur (3) générant un signal indiquant les accélérations d'au moins une partie du corps dudit sujet (B), une unité de commande appropriée pour traiter ledit signal et des moyens de communication pour envoyer un appel à l'aide à une station de commande à distance, dans lequel ladite unité de commande est programmée pour activer automatiquement lesdits moyens de communication uniquement lorsqu'une chute du sujet et son immobilité successive ont été détectées en fonction dudit traitement, **caractérisé en ce que** ladite unité de commande est programmée pour traiter ledit signal en ;
- comptant le nombre de pics d'accélération dépassant une valeur de seuil de pic déterminée dans l'unité de temps ;
- détectant une rotation successive de l'axe de mesure dudit capteur (3) ;
- détectant successivement un signal d'accélération nul pendant une certaine période de temps, qui indique que ledit sujet (B) est immobile.

2. Appareil selon la revendication 1, **caractérisé en ce qu'**il comprend en outre des moyens (6) pour annuler ou confirmer un appel à l'aide, avant ou après que l'appel à l'aide a été envoyé.

3. Appareil selon la revendication 1 ou 2, **caractérisé en ce que** ladite unité de commande comprend une minuterie intégrée détectant le temps écoulé depuis ladite détection de chute.

4. Appareil selon la revendication 3, **caractérisé en ce que** lesdits moyens de communication peuvent être désactivés par ledit sujet afin d'empêcher l'envoi de l'appel à l'aide dans un intervalle de temps prédéterminé tel que mesuré par ladite minuterie.

5. Appareil selon l'une quelconque des revendications 1 à 4 précédentes, **caractérisé en ce qu'**il comprend un signaleur (5) générant un signal d'alarme préalable pour ledit sujet (B) avant d'envoyer ledit appel à l'aide.

6. Appareil selon l'une quelconque des revendication 1 à 5 précédentes, **caractérisé en ce que** ledit capteur (3) est un accéléromètre biaxial.

7. Appareil selon l'une quelconque des revendications 1 à 6 précédentes, **caractérisé en ce qu'**il comprend en outre un ou plusieurs capteurs choisis parmi un capteur de température (7) approprié pour détecter la température du corps dudit sujet et un capteur de découplage (8) approprié pour détecter que l'appareil a été séparé dudit sujet (B).

8. Appareil selon l'une quelconque des revendications 1 à 7 précédentes, **caractérisé en ce que** les moyens de communication comprennent une interface pour le raccordement au réseau de téléphone GSM.

9. Procédé pour envoyer un appel à l'aide suite aux chutes et à l'immobilité d'un sujet (B), comprenant les étapes consistant à mesurer les accélérations d'au moins une partie du corps dudit sujet, comparer lesdites accélérations avec le temps avec des valeurs de référence afin d'identifier une chute et l'immobilité du sujet et l'étape consistant à envoyer un appel à l'aide lorsque cela est nécessaire en fonction de ladite comparaison, ledit appel à l'aide étant envoyé d'une manière automatique, **caractérisé en ce que** ladite étape de comparaison comprend les étapes consistant à :
- compter le nombre de pics d'accélération dépassant une valeur de seuil de pic déterminée dans l'unité de temps ;
- détecter si une rotation successive dudit sujet (B) s'est produite ;
- détecter, lorsque ladite rotation s'est produite, si l'accélération est nulle pendant un certain temps, ledit appel à l'aide automatique étant envoyé après un intervalle de temps prédéterminé à partir du moment où ladite chute et ladite immobilité ont été détectées en fonction de ladite comparaison.

10. Procédé selon la revendication 9, **caractérisé en ce que** ladite étape consistant à envoyer l'appel à l'aide automatique peut être activée manuellement par ledit sujet ou peut être arrêtée manuellement pendant ou après ledit intervalle de temps prédéterminé.

11. Procédé selon la revendication ou 10, **caractérisé en ce qu'**il comprend en outre l'étape consistant à générer un signal d'alarme préalable pour informer ledit sujet que l'appel à l'aide vient d'être envoyé.

12. Procédé selon l'une quelconque des revendications 9 à 11 précédentes, **caractérisé en ce qu'**il comprend en outre l'étape consistant à mesurer la température de l'environnement autour dudit sujet.
